**Europäisches Patentamt**

**European Patent Office**

**Office européen des brevets**

(19)

(11) Veröffentlichungsnummer : **0 103 793 B1**

# (12) EUROPÄISCHE PATENTSCHRIFT

(45) Veröffentlichungstag der Patentschrift :
**17.08.88**

(21) Anmeldenummer : **83108626.9**

(22) Anmeldetag : **01.09.83**

(51) Int. Cl.⁴ : **A 61 N  1/26**, A 61 N  1/04, A 61 H  9/00

(54) Saugeinrichtung zur Applikation von Saugelektroden und zur Saugmassage.

(30) Priorität : **22.09.82 DE 3235025**

(43) Veröffentlichungstag der Anmeldung :
**28.03.84 Patentblatt 84/13**

(45) Bekanntmachung des Hinweises auf die Patenterteilung : **17.08.88 Patentblatt 88/33**

(84) Benannte Vertragsstaaten :
**CH GB LI NL**

(56) Entgegenhaltungen :
**DE-A- 2 400 763**
**DE-A- 2 949 001**
**DE-B- 1 224 847**
**DE-B- 1 491 571**
**DE-B- 2 365 008**
**Firmenprospekte Vacotron 423 und Endomed 422 der Firma ENRAF-NONIUS, Delft (NL); Produktionformation jono-modulator automatik und vacufir plus der Firma mela Elektromedizin, München (DE); Firmenprospekt VACOMED 3 der Firma Bosch Medizintechnik**

(73) Patentinhaber : **Erbe Elektromedizin GmbH.**
**Ebertstrasse 35**
**D-7400 Tübingen (DE)**

(72) Erfinder : **Binder, Karl-Heinz**
**Wiesenstrasse 7**
**D-7407 Rottenburg 5 (DE)**
Erfinder : **Farin, Günter**
**Kapellenweg 15**
**D-7400 Tübingen-Hirschau (DE)**
Erfinder : **Fischer, Klaus**
**Immengasse 1**
**D-7270 Nagold-Emmingen (DE)**
Erfinder : **Göttle, Ferdinand**
**Hohenlaienstrasse 6**
**D-7457 Bissingen (DE)**

(74) Vertreter : **Endlich, Fritz, Dipl.-Phys.**
**Postfach 1326 Blumenstrasse 8**
**D-8034 Germering (DE)**

Jouve, 18, rue St-Denis, 75001 Paris, France

**Beschreibung**

Die Erfindung betrifft eine Saugeinrichtung zur Applikation von Saugelektroden und zur Saugmassage entsprechend den Oberbegriffen der Patentansprüche 1 und 2.

In der Elektrotherapie, z. B. Interferenzstrom-Therapie nach NEMEC oder Elektrotherapie mit diadynamischen Strömen nach BERNARD, werden seit mehreren Jahren Saugelektroden entsprechend DE-PS 12 24 847 angewendet. Hierzu sind inzwischen viele Saugeinrichtungen bekannt, die entweder als spezielles Sauggerät oder als Baugruppe in verschiedenen Elektrotherapiegeräten integriert sind.

Diese bekannten Saugeinrichtungen bestehen in der Regel aus einer elektromotorisch betriebenen Vakuumpumpe, an welche die bekannten Saugelektroden über Rohr- und Schlauchleitungen so angeschlossen sind, daß innerhalb dieser Saugelektroden ein ausreichender Unterdruck entsteht, so daß die Saugelektroden auf der Haut des Patienten haften. Um zu verhindern, daß der Unterdruck innerhalb der Saugelektroden so groß wird, daß der Patient Schmerzen empfindet oder daß an den Saugstellen unter der Haut des Patienten Hämatome entstehen, kann bei bekannten Saugeinrichtungen der Unterdruck auf beliebige Werte zwischen ca. 0 und — 0,7 bar eingestellt werden. Diese Einstellung erfolgt beispielsweise durch einen einstellbaren Bypaß, der parallel zu den Saugelektroden angeordnet ist und durch den so viel Luft strömt, daß der gewünschte Unterdruck infolge der begrenzten Saugleistung der Vakuumpumpe erreicht wird. Entsteht bei diesen bekannten Saugeinrichtungen während der Behandlung eine Undichtigkeit in den Saugleitungen oder, was erfahrungsgemäß relativ oft vorkommt, eine Undichtigkeit zwischen der Haut des Patienten und der Saugelektrode, so fällt der Unterdruck entsprechend der Luftmenge, welche durch diese undefinierten Undichtigkeiten in die Saugeinrichtung strömt, ab. Die Folge hiervon ist, daß die Saugelektroden nicht am Patienten haften oder während der Behandlung plötzlich vom Patienten abfallen. Besonders problematisch ist dieser unerwünschte Nebeneffekt während des Anlegens des Saugelektroden an den Patienten. Hierbei ist es insbesondere bei der Interferenzstrom-Therapie, bei welcher vier Saugelektroden am Patienten angelegt werden müssen, in der Regel nicht möglich, alle Saugelektroden gleichzeitig am Patienten anzulegen, so daß während des Anlegens der ersten Saugelektrode die anderen Saugelektroden noch völlig offen sind und durch diese Luft in die Saugeinrichtung strömen kann. Um zu gewährleisten, daß die erste am Patienten angelegte Saugelektrode am Patienten haften bleibt während die anderen Saugelektroden noch nicht am Patienten angelegt sind, muß der oben genannte Bypaß der Saugeinrichtung während des Anlegens der ersten Saugelektroden so weit geschlossen werden, daß ein ausreichender Unterdruck innerhalb dieser ersten angelegten Saugelektroden entstehen kann. Je mehr Saugelektroden am Patienten angelegt sind, desto weniger Luft kann durch die restlichen noch nicht angelegten Saugelektroden in die Saugeinrichtung strömen, wodurch der Unterdruck mit der Zahl der angelegten Saugelektroden ansteigt. Auf diese Weise kann der Unterdruck insbesondere nach dem Anlegen der letzten Saugelektrode so stark ansteigen, daß der Patient Schmerzen empfindet oder die Gefahr besteht, daß an den Saugstellen unter der Haut des Patienten Hämatome entstehen. Um letzteres zu verhindern, empfehlen einige Hersteller bekannter Saugeinrichtungen, mit dem Fortschreiten der Anlage der Saugelektroden an den Patienten den Unterdruck an dem hierfür vorgesehenen Bypaß jeweils auf den erforderlichen Wert zu reduzieren.

Besonders schwierig ist bei bekannten Saugeinrichtungen die Anlage der Saugelektroden an den Patienten während der sog. Saugmassage. Hierbei wird bei bekannten Saugeinrichtungen, siehe z. Bsp. Produktinformation Vakuum-Zusatzgerät VACUFIX PLUS der Firma mela Elektromedizin, München, 1973, zusätzlich zu dem oben erwähnten Bypaß zur Einstellung des Unterdruckes ein weiteren Bypaß parallel geschaltet, welcher durch ein Magnetventil periodisch geöffnet und geschlossen wird, so daß der Unterdruck innerhalb der Saugelektroden periodisch zwischen einem oberen und einem unteren Wert pulsiert. Da sich der obere und der untere Wert des Unterdruckes bei bekannten Saueinrichtungen mehr oder weniger undefiniert aus der Saugleistung der Vakuumpumpe und der durch die Bypässe und Undichtigkeiten strömenden Luft einstellen, kommt es bei bekannten Saugeinrichtungen relativ oft vor, daß die Saugelektroden während der Saugmassage vom Patienten abfallen.

Es ist Aufgabe der Erfindung, bekannte Saugeinrichtungen der genannten Art derart zu verbessern, daß sowohl für die Saugmassage als auch für die Applikation mit möglichst einfachen Mitteln eine genauere Aufrechterhaltung des maximalen und des minimalen Unterdrucks möglich ist, so daß die Gefahr des Abfallens der Saugelektroden verringert wird, ohne daß bei deren Applikation ein für den Patienten nachteilig hoher Unterdruck auftreten kann.

Diese Aufgabe wird durch den Gegenstand des Patentanspruchs 1 beziehungsweise den Gegenstand des Patentanspruchs 2 gelöst. Vorteilhafte Weiterbildungen der Erfindung sind Gegenstand der Unteransprüche.

Sowohl bei der Lösung mit elektrischer Steuerung entsprechend Patentanspruch 1 als auch bei der Lösung mit mechanischer Steuerung entsprechend Patentanspruch 2 können der für die Applikation der Saugelektroden erforderliche Unterdruck sowie die für die Saugmassage erforderlichen unterschiedlichen Unterdrucke an Sollwertgebern unabhängig voneinander bereits vor der Applikation und vor der Saugmassage reproduzierbar voreingestellt werden. Ferner wird während der Applikation und der Saugmassage unabhängig von der Anzahl der jeweils verwendeten Saugelektroden auf die vorher eingestellten Werte automatisch geregelt.

2

In vorteilhafter Weiterbildung der Lösung mit einer elektrischen Steuerung ist es ferner möglich, die Vakuumpumpe nicht nur in Dauerbetrieb zu verwenden, wie es bei bekannten Saugeinrichtungen der Fall ist, sondern auch einen Aussetzbetrieb für die Vakuumpumpe vorzusehen. Hierdurch kann das Pumpengeräusch und die Vibration der Saugeinrichtung wesentlich verringert werden. Zur Vermeidung hoher Stromspitzen bei mehrmaligen Einschaltungen des Elektromotors der Vakuumpumpe während einer Behandlung erfolgt dann die Einschaltung des Elektromotors zweckmäßigerweise über eine elektronische Schaltung, welche die Betriebswechselspannung stets nur im Nullpunkt auf die Erregerwicklung des Motors schaltet. Praktische Erprobungen einer derartigen Saugeinrichtung haben gezeigt, daß die Vakuumpumpe bei fehlerfreier Applikation der Saugelektroden am Patienten weniger als 5 % der Behandlungsdauer eingeschaltet ist, während sie bei bekannten Saugeinrichtungen während der gesamten Behandlungsdauer eingeschaltet sein muß. Insbesondere hat sich bei diesen praktischen Erprobungen gezeigt, daß es von extremen Ausnahmefällen abgesehen praktisch nicht mehr vorkommt, daß die Saugelektroden während der Saugmassage vom Patienten abfallen.

Anhand der Zeichnung soll die Erfindung beispielsweise näher erläutert werden.

Figur 1 zeigt ein Ausführungsbeispiel mit pneumatischer Regelung des Unterdruckes für die Applikation des Saugelektroden bzw. der beiden unabhängig voneinander einstellbaren Unterdrucke für die Saugmassage.

Figur 2 zeigt ein Ausführungsbeispiel mit elektropneumatischer Regelung des Unterdruckes zur Applikation der Saugelektroden bzw. der beiden voneinander unabhängig einstellbaren Unterdrucke für die Saugmassage, wobei außerdem die Vakuumpumpe nicht ständig eingeschaltet ist.

Figur 3 zeigt die elektrische Steuerung für das Ausführungsbeispiel in Figur 2.

Figur 4 bis 7 zeigen grafische Darstellungen zur Erläuterung der Wirkungen der vier Komparatoren in Figur 3.

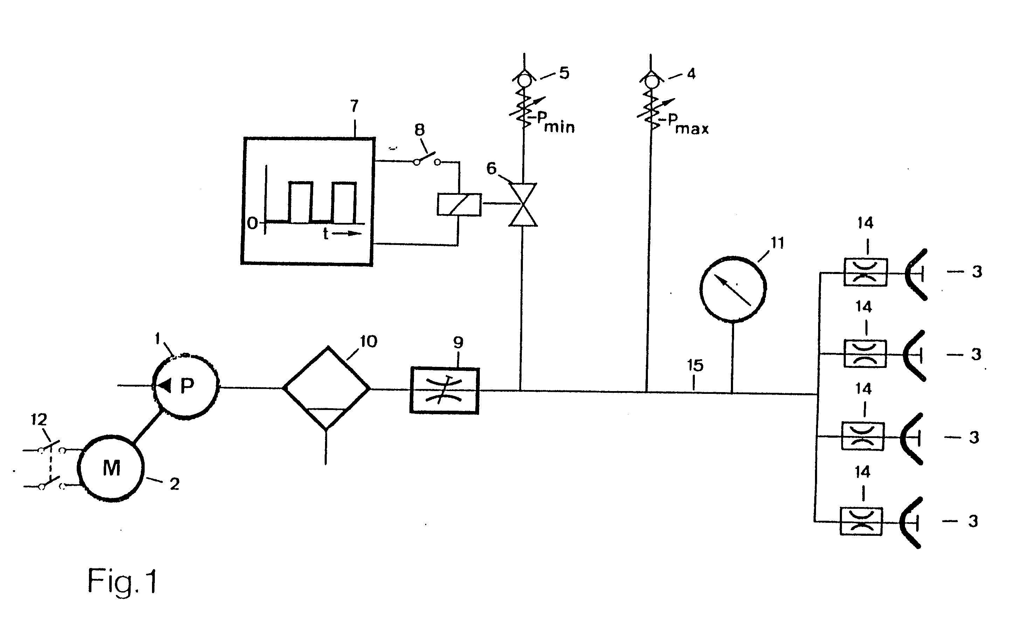

Die in Figur 1 dargestellte Saugeinrichtung besteht aus einer Vakuumpumpe 1, welche vom Elektromotor 2 angetrieben wird sobald und solange der Schalter 12 geschlossen ist und Betriebsspannung an dem Elektromotor 2 anliegt. Dadurch steigt der Unterdruck in der Saugleitung 15 so weit an, bis der an dem Ventil 4 eingestellte Wert erreicht ist, wobei dieses Ventil 4 so weit und so lange öffnet, daß die hierdurch einströmende Luft ein weiteres Ansteigen des Unterdruckes in der Saugleitung 15 verhindert. Der Wert des Unterdruckes in der Saugleitung 15 wird durch das Vakuummeter 11 angezeigt. Um zu verhindern, daß die durch die noch nicht am Patienten angelegten Saugelektroden 3 in die Saugleitung 15 strömende Luft den Unterdruck zu stark verringert, sind vor jeder Saugelektrode 3 Drosselventile 14 eingeschaltet, welche den Luftstrom so weit drosseln, daß der Unterdruck in der Saugleitung 15 mindestens — 0,1 bar erreicht, wenn keine Saugelektrode 3 am Patienten angelegt ist. Mit zunehmender Anzahl der am Patienten angelegten Saugelektroden 3 steigt der Wert des Unterdruckes in der Saugleitung 15 an. Er kann jedoch nicht höher ansteigen, als am Ventil 4 eingestellt ist. Die Drosselventile 14 sollen also verhindern, daß der Unterdruck in der Saugleitung 15 bei laufender Vakuumpumpe unter einen Minimalwert von 0,1 bar abfällt, während das Ventil 4 verhindert, daß der hieran eingestellte Maximalwert des Unterdruckes überschritten wird.

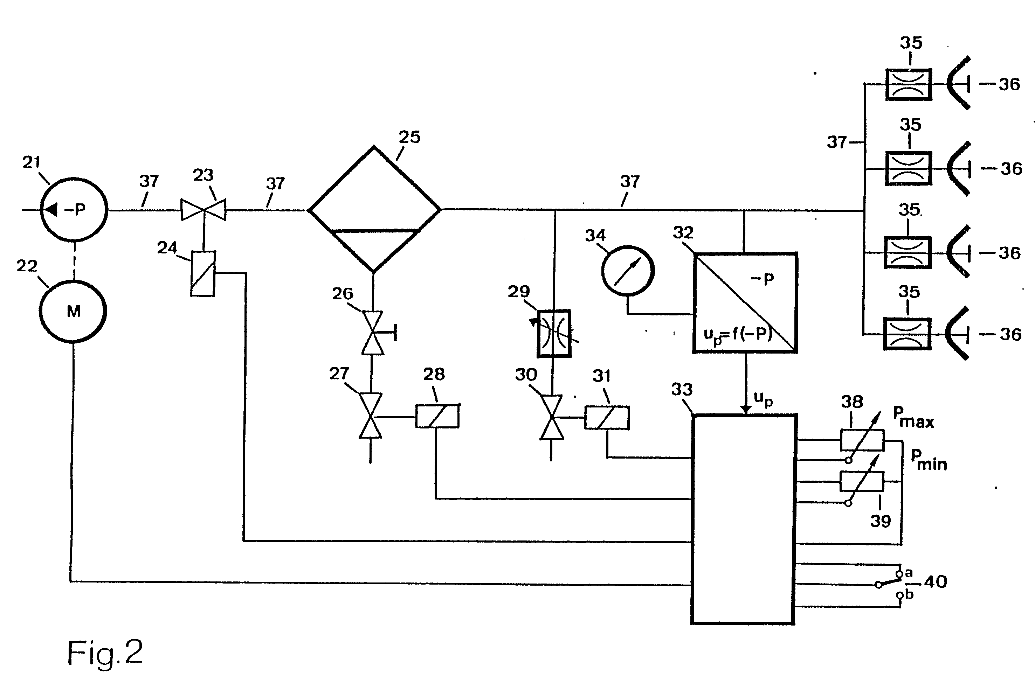

Für die Saugmassage ist parallel zum Ventil 4 ein weiteres Ventil 5 geschaltet, welches ebenfalls ab einem einstellbaren Wert des Unterdruckes, der geringer sein muß als der am Ventil 4 eingestellte Wert, öffnet und immer dann, wenn das Magnetventil 6 geöffnet ist, so viel Luft in die Saugleitung 15 strömen läßt, daß der am Ventil 5 eingestellte Unterdruck eingehalten wird. Das Magnetventil 6 wird von einem elektronischen Intervallschalter 7 periodisch geöffnet und geschlossen, wenn der Schalter 8 für die Saugmassage geschlossen ist. Die Periodendauer des Intervallschalters 7 kann stufenlos oder in vorgegebenen Stufen verändert werden. Das Magnetventil 6 bleibt geschlossen, wenn der Schalter 8 geöffnet ist. Das justierbare Drosselventil 9 verhindert zusammen mit dem Volumen des Wasserabscheiders 10, daß die Pulsation des Unterdruckes hinter der Vakuumpumpe 1 in die Saugleitung 15 gelangt und dort Schwingen bzw. Flattern der Ventile 4 und 5 verursacht. Ein zweites Ausführungsbeispiel soll anhand der Figuren 2 bis 7 beschrieben werden.

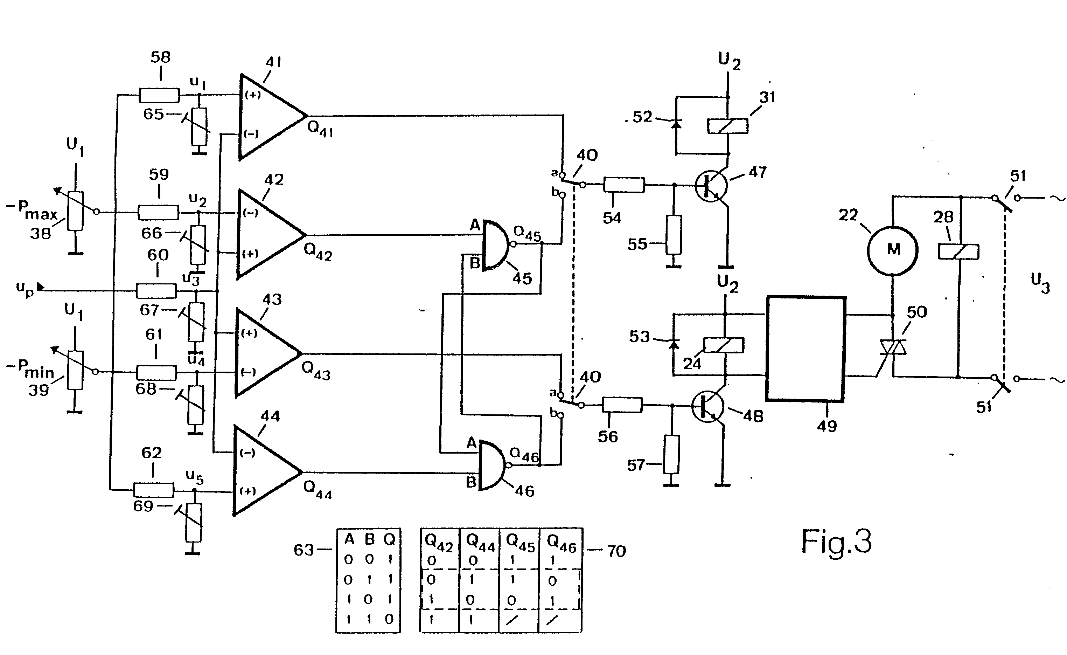

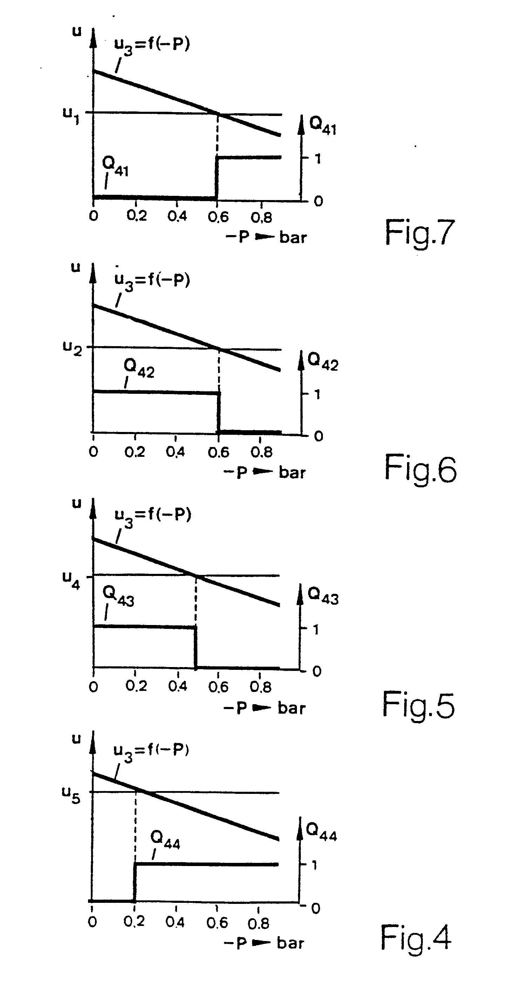

In Figur 2 sind die erfindungsrelevanten Details des zweiten Ausführungsbeispieles schematisch dargestellt, wobei die elektronische Steuerung 33 in Figur 3 ausführlicher beschrieben wird. In den Figuren 4 bis 7 sind die Wirkungen der vier Komparatoren 41 bis 44 graphisch dargestellt.

Mit Rücksicht auf eine möglichst übersichtliche Beschreibung des zweiten Ausführungsbeispieles wird sie beim Ausführungsbeispiel 1 zuerst die Funktion der Saugeinrichtung bei der Applikation der Saugelektroden am Patienten ohne Saugmassage und anschließend die Funktion der Saugmassage beschrieben.

Bei der Applikation der Saugelektroden am Patienten soll der Unterdruck innerhalb der Saugelektroden einerseits so groß sein, daß die Saugelektroden sicher auf der Haut des Patienten haften, andererseits aber nicht so groß werden, daß der Patient Schmerzen erleidet oder gar Hämatome unter dessen Haut entstehen.

Es ist daher erforderlich, daß der Unterdruck innerhalb der Saugelektroden 36 individuell eingestellt werden kann und dieser eingestellte Wert des Unterdruckes während der gesamten Behandlung möglichst konstant bleibt. Hierfür ist an die Saugleitung 37 ein Drucksensor 32 angeschlossen, welcher den Unterdruck in eine dem Unterdruck proportionale elektrische Spannung $u_p = f(— P)$ umwandelt. Diese Spannung $u_p$ wird über den Widerstand 60 dem Komparator 43, aber auch den weiter unten aufgeführten Komparatoren 41, 42 und 44, zugeführt. Mittels des Trimmwiderstandes 67 kann diese Spannung mit Rücksicht auf die Toleranzen der Bauelemente auf den erforderlichen Wert $u_3$ eingestellt werden. Diese Spannung $u_3$ wird auf den nicht invertierenden Eingang (+) des Komparators 43 geführt. Auf den invertierenden Eingang (—) des Komparators 43 wird die Spannung $u_4$ geführt, die aus einer Konstantspannungsquelle $U_1$ abgeleitet und über das Potentiometer 39 kontinuierlich, oder über einen Stufenschalter in Stufen einstellbar ist. Über den Widerstand 61 und den Trimmwiderstand 68 kann die Spannung $u_4$ justiert werden. In Figur 5 ist die Abhängigkeit des Ausgangssignales $Q_{43}$ des Komparators 43 als Funktion der Eingangssignale $u_3$ and $u_4$ dargestellt. Ist $u_3$ größer als $u_4$, so ist das Ausgangssignal $Q_{43}$ des Komparators 43 $Q_{43} = 1$. Das Ausgangssignal $Q_{43} = 1$ schaltet den Transistor 48 in den leitenden Zustand, wenn der Umschalter 40 in Stellung a geschaltet ist. Hierdurch wird sowohl die Magnetspule 24 erregt und dadurch das Magnetventil 23 geöffnet als auch der Elektromotor 22, welcher die Vakuumpumpe 21 antreibt eingeschaltet. Die Einschaltung des Elektromotors 22 erfolgt hierbei über den Triac 50 welcher wiederum von der Triaczündung 49 gezündet wird. Diese Triaczündung 49 besteht aus einem Optokoppler, welcher die Betriebsspannung $U_2$ des Transistors 48 von der Betriebsspannung $U_3$ des Elektromotors 22 isoliert, einem Nullpunktdetektor, welcher den Triac stets nur im Nulldurchgang der Betriebswechselspannung $U_3$ zündet, wodurch hohe Stromimpulse beim wiederholten Einschalten des Elektromotores vermieden werden und einem Diac, welcher in die Ansteuerung des Gates des Triacs geschaltet ist. Da derartige Triaczündungen als integrierte Schaltung im Handel erhältlich sind, ist die Triaczündung 49 in Figur 3 nicht ausführlich sondern als Block dargestellt. Mit steigendem Unterdruck in der Saugleitung 37 wird die Spannung $u_3 = f(— P)$ kleiner. Wird die Spannung $u_3$ kleiner als die Spannung $u_4$, so springt das Ausgangssignal $Q_{43}$ von $Q_{43} = 1$ auf $Q_{43} = 0$. Hierdurch wird der Transistor 48 gesperrt, das Magnetventil 23 geschlossen und der Elektromotor 22 der Vakuumpumpe 21 abgeschaltet. Der Elektromotor 22 und damit die Vakuumpumpe 21 bleiben solange abgeschaltet, bis der Unterdruck in der Saugleitung 37 so weit abgefallen ist, daß $u_3$ wieder größer wird als $u_4$. Dies kann während der Applikationsdauer einerseits durch Undichtigkeiten der Saugleitung 37 oder der Saugelektroden 36, andererseits durch Verkleinerung der Spannung $u_4$ am Potentiometer 39 erfolgen, wenn der Unterdruck während der Applikation höher eingestellt wird.

Um zu realisieren, daß der Unterdruck innerhalb der Saugelektroden 36 während der Applikation durch Einstellen der Spannung $u_4$ am Potentiometer 39 nicht nur erhöht, sondern auch schnell verringert werden kann, ist der Komparator 41 vorgesehen. Dieser Komparator 41 ist am Trimmerwiderstand 65 so eingestellt, daß die Eingangsspannung $u_1$ des Komparators 41 etwas kleiner ist als die Eingangsspannung $u_4$ des Komparators 43. Wird die Eingangsspannung $u_3$ des Komparators 41 kleiner als die Eingangsspannung $u_1$, so liefert der Komparator 41, wie in Figur 7 dargestellt, das Ausgangssignal $Q_{41} = 1$. Hierdurch wird, wenn der Umschalter 40 in Stellung a geschaltet ist, der Transistor 47 leitend geschaltet und öffnet über die Magnetspule 31 das Magnetventil 30, so daß die Saugleitung 37 so lange über das Drosselventil 29 belüftet wird, bis das Eingangssignal $u_3$ des Komparators 41 wieder größer ist als das Eingangssignal $u_1$. Sobald $Q_{41} = 0$ wird, schließt das Magnetventil 30. Die Regelgenauigkeit des Unterdruckes in der Saugleitung 37 während der Applikation ist von der Differenz zwischen den Eingangsspannungen $u_1$ und $u_4$ abhängig, die durch Justage der Trimmwiderstände 65 und 68 beliebig klein eingestellt werden kann. Sind die Undichtigkeiten der Saugleitung 37 und der Saugelektroden 36 gering, was in der Praxis durchaus erreichbar ist, so läuft der Elektromotor der Vakuumpumpe nur während des Einschaltens der Saugenrichtung und hin und wieder kurz während der Applikationsdauer, um die durch Restleckstellen in die Saugleitung gelangte Luft wieder herauszupumpen.

Zur Durchführung der Saugmassage wird der Umschalter 40 in die Schaltstellung b geschaltet. In dieser Schaltstellung sind die während der reinen Applikation benutzten Komparatoren 41 und 43 außer Funktion. Während der Saugmassage wirken die Komparatoren 42 und 44, welche auf das aus den NAND-Gattern 45 und 46 bestehende RS-Flipp-Flopp geschaltet sind.

Während der Saugmassage soll der Unterdruck innerhalb der Saugelektroden 36 zwischen einem einstellbaren unteren Wert $P_{min}$ und einem ebenfalls einstellbaren oberen Wert $P_{max}$ periodisch hin un her pulsieren. Der untere Wert $P_{min}$ wird über das Potentiometer 39 eingestellt, wodurch die Eingangsspannung $u_5$, welche außerdem am Trimmwiderstand 69 justiert werden kann, des Komparators 44 variiert. Die Abhängigkeit des Ausgangssignales $Q_{44}$ von den Eingangssignalen $u_3$ und $u_5$ ist in Figur 4 dargestellt. Der obere Wert $P_{max}$ wird über das Potentiometer 38, welches ebenfalls wie das Potentiometer 39 aus einer Konstantspannungsquelle $U_1$ versorgt wird, eingestellt, wodurch die Eingangsspannung $u_2$, welche außerdem am Trimmwiderstand 66 justiert werden kann, des Komparators 42 variiert wird.

Die Abhängigkeit des Ausgangssignales $Q_{42}$ von den Eingangsspannungen $u_3$ und $u_2$ ist in Figur 6 dargestellt. Die Abhängigkeit der Ausgangssignale Q der NAND-Gatter 45 und 46 von deren Eingangssignalen A und B sind in der Matrix 63 dargestellt. Die Abhängigkeit der Ausgangssignale $Q_{45}$ und $Q_{46}$ aus den NAND-Gattern 45 und 46 in Abhängigkeit von den Eingangssignalen $Q_{42}$ und $Q_{44}$ aus den Komparatoren 42 und 44 sind in der Matrix 70 dargestellt. Wie aus Figur 4 und 6 erkennbar, ist der Zustand $Q_{42} = 0$ und $Q_{44} = 0$ bei eingeschalteter Saugeinrichtung nicht vorgesehen und braucht deshalb in dieser Schaltung nicht beachtet zu werden. Auch der Zustand $Q_{42} = 1$ und $Q_{44} = 1$, der in dieser Schaltung vorkommt, kann vernachlässigt werden, weil das RS-Flipp-Flopp nur auf Eingangssprünge von 1 nach 0 gesetzt und zurückgesetzt werden kann, nicht jedoch bei Eingangssprüngen von 0

4

nach 1.

Kurz nach Einschalten der Saugeinrichtung aus dem Zustand —P = 0 ist, wie aus den Figuren 4 und 6 erkennbar, $Q_{42} = 1$ und $Q_{44} = 0$. Das RS-Flipp-Flopp liefert in diesem Zustand die Ausgangssignale $Q_{45} = 0$ und $Q_{46} = 1$. Hierdurch wird, identisch wie oben bei der reinen Applikation beschrieben, über den Transistor 48 und die Zündschaltung 49 sowie den Triac 50 der Elektromotor 22 der Vakuumpumpe 21 eingeschaltet. Der Unterdruck in der Saugleitung 37 steigt stetig an. Bei beispielsweise 0,2 bar springt das Ausgangssignal $Q_{44}$ des Komparators 44 von 0 auf 1, was jedoch, wie oben beschrieben, ohne Wirkung auf den Zustand des RS-Flipp-Flopps bleibt. Erreicht der Unterdruck beispielsweise 0,6 bar, so springt das Ausgangssignal $Q_{42}$ des Komparators 42 von 1 nach 0, wodurch das RS-Flipp-Flopp von $Q_{45} = 0$ und $Q_{46} = 1$ auf $Q_{45} = 1$ und $Q_{46} = 0$ zurückgesetzt wird. Hierdurch wird der Elektromotor 22 der Vakuumpumpe 21 abgeschaltet und gleichzeitig das Magnetventil 30 über den Transistor 47 geöffnet, wodurch die Saugleitung 37 und die Saugelektroden 36 über das Drosselventil 29 belüftet wird. Hierbei sinkt der Unterdruck innerhalb der Saugleitung 37 und den Saugelektroden 36 je nach Einstellung des Drosselventiles 29 mehr oder weniger schnell ab. Bei beispeilsweise —P = 0,6 bar springt das Auggangssignal $Q_{42}$ des Komparators 42 von 0 nach 1, was jedoch ohne Wirkung auf den Schaltzustand des RS-Flipp-Flopps bleibt. Erreicht der Unterdruck beispielsweise —P = 0,2 bar, so springt das Ausgangssignal $Q_{44}$ von 1 nach 0, wodurch die Ausgangssignale des RS-Flipp-Flopps von $Q_{45} = 1$ und $Q_{46} = 0$ wieder auf $Q_{45} = 0$ und $Q_{46} = 1$ zurückgesetzt werden. Hierdurch wird der Elektromotor 22 der Vakuumpumpe 21 eingeschaltet und das Magnetventil 30 geschlossen. Dieser Zyklus wiederholt sich beliebig oft, so daß der Unterdruck beispielsweise zwischen —P = 0,2 bar und —P = 0,6 bar auf und ab pulsiert. Die Zyklusdauer kann am Drosselventil 29 eingestellt werden, wobei die Belüftungsgeschwindigkeit der Saugleitung 37 und der Saugelektroden 36 beeinflußt wird.

Um zu verhindern, daß Wasser aus den innerhalb der Saugelektroden in bekannter Weise verwendeten mit Wasser getränkten Schwämmen in die Vakuumpumpe gelangen kann, ist in die Saugleitung 37 ein Wasserabscheider 25 angeordnet. Dieser Wasserabscheider 25 muß regelmäßig entleert werden. Hierfür ist ein manuell betätigbares Ventil 26 vorgesehen. Um zu verhindern, daß der Unterdruck innerhalb der Saugleitung 37 und der Saugelektroden 36 während der Anwendung der Saugeinrichtung zusammenbricht wenn dieses Ventil 26 geöffnet wird, ist diesem Ventil 26 ein Magnetventil 27 in Serie geschaltet. Die Magnetspule 28 dieses Magnetventiles 27 ist parallel zum Netzschalter 51 der Saugeinrichtung geschaltet, und zwar in der Art, daß das Magnetventil 27 stets geschlossen ist, wenn die Saugeinrichtung über den Netzschalter 51 eingeschaltet ist, und geöffnet ist, wenn die Saugeinrichtung über den Netzschalter 51 abgeschaltet ist.

Die Verwendung des Drucksensors 32, welcher den Wert des Unterdruckes in der Saugleitung in eine proportionale elektrische Spannung umwandelt, bietet außerdem die Möglichkeit, den Wert des Unterdruckes auf einem elektrischen Meßinstrument 34 statt auf einem konventionellen Manometer anzuzeigen. Dies ist insbesondere deshalb vorteilhaft, weil es bisher kein preiswertes Manometer gibt, welches der modernen Formgestaltung elektromedizinischer Geräte gerecht wird. In bekannten Saugeinrichtungen werden, wenn überhaupt, runde Manometer verwendet, wie sie aus dem Dampfmaschinen-Zeitalter bekannt sind.

Das Magnetventil 23, das nur dann geöffnet ist, wenn die Vakuumpumpe 21 arbeitet und sofort schließt, wenn die Vakuumpumpe abgeschaltet wird, soll verhindern, daß der Unterdruck innerhalb der Saugleitung 37 und der Saugelektroden 36 durch Undichtigkeiten in der Vakuumpumpe zu schnell wieder zusammenbricht. Außerdem verhindert dieses Magnetventil, daß der Unterdruck nach Abschalten der Vakuumpumpe bei Erreichen des eingestellten Sollwertes weiter ansteigt, weil die Vakuumpumpe infolge der Schwungmassen des Elektromotores und der rotierenden Teile der Vakuumpumpe relativ lange ausläuft. Bei Verwendung leckdichter Vakuumpumpen, welche außerdem nach dem Abschalten sofort still stehen und nicht während der Auslaufdauer weiterpumpen, kann auf dieses Magnetventil 23 verzichtet werden.

Die Dioden 52 und 53 in Figur 3 verhindern hohe elektrische Spannungsspitzen beim Abschalten der Magnetspulen 31 und 24.

**Patentansprüche**

1. Saugeinrichtung mit mehreren Saugelektroden (36) für eine Saugmassage während einer Elektrotherapie mit nieder- oder mittelfrequenten Reizströmen, bei der die Saugelektroden über eine gemeinsame Saugleitung (37) mit einer Vakuumpumpe (21) in Verbindung stehen, bei der eine Belüftungseinrichtung mit der Saugleitung verbunden ist, durch die die Saugleitung bei der Applikation der Saugelektroden belüftbar ist, und bei der für die Saugmassage eine Einrichtung zur Erzeugung eines pulsierenden Unterdrucks vorgesehen ist, dadurch gekennzeichnet, daß an die gemeinsame Saugleitung (37) ein Drucksensor (32) angeschlossen ist, welcher den Wert (—P) des Unterdrucks in eine elektrische Spannung $u_p = f (—P)$ umwandelt, daß eine elektrische Steuerung (33) mit einem ersten Sollwertgeber (39) vorgesehen ist, an dem ein gewünschter Grenzwert des Unterdrucks für die Applikation der Saugelektroden (36) einstellbar ist, daß die elektrische Steuerung (33) einen zweiten Sollwertgeber (38) aufweist, an dem für die Saugmassage ein zweiter Grenzwert des Unterdrucks einstellbar ist, und daß die elektrische Steuerung (33) an die Belüftungseinrichtung (30, 31) angeschlossen ist, so daß der sowohl für die Applikation als auch für die Saugmassage erforderliche Unterdruck auf die vor der Applikation der Saugelektroden eingestellten Sollwerte begrenzbar oder regelbar ist.

2. Saugeinrichtung mit mehreren Saugelektroden (3) für eine Saugmassage während einer Elektrotherapie mit nieder- oder mittelfrequenten Reizströmen, bei der die Saugelektroden über eine gemeinsame Saugleitung (15) mit einer Vakuumpumpe (1) in Verbindung stehen, bei der eine erste Ventileinrichtung (4) mit der Saugleitung verbunden ist, durch die die Saugleitung bei der Applikation der Saugelektroden belüftbar ist, und bei der eine zweite Ventileinrichtung (16) mit der Saugleitung verbunden ist, durch deren Betätigung für die Saugmassage der Unterdruck zwischen einem minimalen und einem maximalen Unterdruck pulsieren kann, dadurch gekennzeichnet, daß für eine automatische Begrenzung des maximalen Unterdrucks ($-P_{max}$) bei der Applikation der Saugelektroden (3) und bei der Durchführung der Saugmassage die erste Ventileinrichtung (4) eine reproduzierbare Voreinstellung ermöglichend derart ausgebildet ist, daß sie beim Erreichen des maximalen Unterdrucks ($-P_{max}$) so weit und so lange öffnet, daß die einströmende Luft ein weiteres Ansteigen des Unterdrucks über den eingestellten maximalen Unterdruck ($-P_{max}$) hinaus verhindert, und daß zur automatischen Begrenzung des minimalen Unterdrucks ($-P_{min}$) für die Saugmassage der zweiten Ventileinrichtung (6) eine dritte Ventileinrichtung (5) zugeordnet ist, an der der minimale Unterdruck ($-P_{min}$) für die Saugmassage voreinstellbar ist.

3. Saugeinrichtung nach Anspruch 1 oder 2, dadurch gekennzeichnet, daß zwischen jeder Saugelektrode (3 ; 36) und der gemeinsamen Saugleitung (15 ; 37) eine Drosselung (14 ; 35) vorgesehen ist, welche Drosselung den Luftstrom so weit drosselt, daß der Unterdruck in der gemeinsamen Saugleitung (15 ; 37) größer als der für die Applikation der Saugelektroden (3 ; 36) mindestens erforderliche Unterdruck $-P_{min}$ ist, wenn keine Saugelektrode (3 ; 36) am Patienten angelegt ist.

4. Saugeinrichtung nach einem der vorhergehenden Ansprüche, dadurch gekennzeichnet, daß zur Entleerung eines zwischen den Saugelektroden (3 ; 36) und der Vakuumpumpe (1 ; 21) vorgesehenen Wasserabscheiders (10 ; 25) ein manuell betätigbares Absperrventil (26) und ein Magnetventil (27, 28) in Serie angeordnet sind, und daß dieses Magnetventil (27, 28) nur dann geöffnet ist, wenn die Saugeinrichtung abgeschaltet ist.

5. Saugeinrichtung nach Anspruch 1, dadurch gekennzeichnet, daß an den Drucksensor (32) ein elektrisches Meßinstrument (34) angeschlossen ist, das von der Ausgangsspannung $u_p = f(-P)$ des Drucksensors versorgt wird und dessen Skala in Druckwerten geeicht ist.

6. Saugeinrichtung nach Anspruch 1, dadurch gekennzeichnet, daß die elektrische Steuerung (33) den Elektromotor (22) der Vakuumpumpe (21) so lange einschaltet, bis ein für die Applikation einstellbarer minimaler Unterdruck ($-P_{min}$) erreicht ist und abschaltet, sobald dieser Unterdruck erreicht wird, und daß die elektrische Steuerung (33) bei Überschreiten des eingestellten minimalen Unterdrucks die durch ein Magnetventil (30, 31) gebildete Belüftungseinrichtung öffnet, so daß die gemeinsame Saugleitung (37) so weit über eine Drossel (29) belüftet wird, bis dieser eingestellte Unterdruck erreicht ist und dieses Magnetventil (30, 31) wieder schließt, sobald dieser eingestellte Unterdurck erreicht wird.

7. Saugeinrichtung nach Anspruch 6, dadurch gekennzeichnet, daß für die Saugmassage als Sollwertgeber zwei Potentiometer (38, 39) vorgesehen sind, und daß die elektrische Steuerung (33) den Elektromotor (22) der Vakuumpumpe (21) so lange einschaltet, bis der maximale Unterdruck $-P_{max}$ erreicht ist und sobald der maximale Unterdurck $-P_{max}$ erreicht wird, den Elektromotor (22) der Vakuumpumpe (21) abschaltet und gleichzeitig das Magnetventil (30, 31) öffnet, so daß die Saugelektroden (36) so weit belüftet werden, bis der vorher eingestellte minimale Unterdruck $-P_{min}$ erreicht ist und dieses Magnetventil (30, 31) sofort wieder schließt, sobald der minimale Unterdruck $-P_{min}$ erreicht wird und gleichzeitig den Elektromotor (22) der Vakuumpumpe (21) wieder einschaltet, wonach sich dieser Zyklus periodisch wiederholt, so daß der Unterdruck zwischen $-P_{min}$ und $-P_{max}$ periodisch alterniert.

8. Saugeinrichtung nach Anspruch 7, dadurch gekennzeichnet, daß an der elektrischen Steuerung (33) ein Umschalter (40) vorhanden ist, an welchem diese elektrische Steuerung (33) alternativ für die Applikation der Saugelektroden (36) ohne Saugmassage an einer ersten Schaltstellung (a) oder für die Saugmassage an einer zweiten Schaltstellung (b) einstellbar ist.

9. Saugeinrichtung nach einem der Ansprüche 6, 7 oder 8, dadurch gekennzeichnet, daß die Belüftungsgeschwindigkeit an der Drossel (29) eingestellt werden kann, wodurch die Periodendauer während der Saugmassage variiert werden kann.

10. Saugeinrichtung nach einem der Ansprüche 1, 5, 6 oder 7, dadurch gekennzeichnet, daß in der gemeinsamen Saugleitung (37) gleich hinter der Vakuumpumpe (21) ein Magnetventil (23) angeordnet ist, das nur dann geöffnet ist, wenn der Elektromotor (22) der Vakuumpumpe (21) eingeschaltet ist und das sofort schließt, wenn der Elektromotor (22) abgeschaltet wird.

11. Saugeinrichtung nach einem der Ansprüche 1, 6 oder 7, dadurch gekennzeichnet, daß der Elektromotor (22) der Vakuumpumpe (21) über einen Triac (50) ein- und ausgeschaltet wird, wobei die Zündung des Triacs (50) aus einer elektronischen Triaczündung (49) erfolgt, welche Zündimpulse stets nur in Nulldurchgang einer Betriebswechselspannung ($U_3$) liefert und welche außerdem einen Optokoppler zur Potentialtrennung zwischen der Betriebswechselspannung und einer Betriebsspannung ($U$) für Transistoren (47, 48) enthält.

12. Saugeinrichtung nach Anspruch 10, dadurch gekennzeichnet, daß die Einstellung der Unterdrucke — $P_{min}$ und — $P_{max}$ für die Saugmassage über zwei separate Potentiometer (38, 39) erfolgt, die aus einer Konstantspannungsquelle ($U_1$) gespeist werden, wobei die an den Potentiometern (38, 39) abgegriffenen elektrischen Spannungen jeweils mittels eines Spannungsteilers (62, 69 und 59, 66) auf zwei variable Werte ($U_5$ und $U_2$) justiert werden können, die jeder für sich auf die Eingänge eines ersten und zweiten Spannungskomparators (44 und 42) geführt werden, so sie mit der aus dem Drucksensor (32) kommenden Spannung ($U_p$), welche ebenfalls mittels eines Spannungsteilers (60, 67) auf eine variable Spannung ($U_3$) justiert werden kann, verglichen werden, wobei ferner die Ausgangssignale $Q_{44}$, $Q_{42}$ der Komparatoren (44, 42) auf ein RS-Flip-Flop, bestehend aus zwei NAND-Gattern (45, 46) geführt werden, deren Ausgangssignale $Q_{45}$, $Q_{46}$ in Abhängigkeit von den Eingangssignalen $Q_{44}$, $Q_{42}$ in der folgenden Matrix (70)

| $Q_{42}$ | $Q_{44}$ | $Q_{45}$ | $Q_{46}$ |
|---|---|---|---|
| 0 | 0 | 1 | 1 |
| 0 | 1 | 1 | 0 |
| 1 | 0 | 0 | 1 |
| 1 | 1 | / | / |

dargestellt sind, und wobei die Ausgangssignale der NAND-Gatter über Transitoren (47, 48) den Elektromotor (22) der Vakuumpumpe (21), das Magnetventil (30, 31) zur belüftung der Saugleitung (37) und das Magnetventil (23, 24) zur Absperrung der Saugleitung (37) zwischen der Vakuumpumpe (21) und dem Wasserabscheider (25) ansteuern.

13. Saugeinrichtung nach Anspruch 10, dadurch gekennzeichnet, daß das für die Einstellung des Unterdrucks (— $P_{min}$) für die Applikation der Saugelektroden (36) vorgesehen Potentiometer (39) aus einer Konstantspannungsquelle ($U_1$) gespeist ist und die Ausgangsspannung dieses Potentiometers (39) mittels Widerstände (61, 68) auf eine variable Spannung ($U_4$) justiert werden kann, welche auf einen Eingang eines ersten Komparators (43) geführt ist und mit der aus dem Drucksensor (32) kommenden Spannung ($U_3$), welche auf den zweiten Eingang des Komparators (43) geführt ist, verglichen wird, wobei das Ausgangssignal ($Q_{43}$) des Komparators (43) über einen Transistor (48) den Elektromotor (22) der Vakuumpumpe (21) und das Magnetventil (23, 24) ansteuert, das in der Saugleitung (37) gleich hinter der Vakuumpumpe (21) angeordnet ist.

14. Saugeinrichtung nach Anspruch 13, dadurch gekennzeichnet, daß für die Applikation der Saugelektroden parallel zum ersten Komparator (43) ein zweiter Komparator (41) geschaltet ist, dessen Eingangsspannung ($U_1$) mittels Widerstände (58, 65) etwas kleiner justiert ist als die justierbare Eingangsspannung ($U_4$) des ersten Komparators (43), so daß das Ausgangssignal ($Q_{41}$) des zweiten Komparators (41) von 0 auf 1 kippt, wenn der Unterdruck (— $P_{min}$) in der Saugleitung (37) um einen kleinen Betrag ($\Delta P_{min}$) höher ansteigt als am Potentiometer (39) eingestellt, wodurch das Magnetventil (30, 31) zum Belüften der Saugleitung über einen Transistor (47) so lange geöffnet wird, bis der Unterdruck (— $P_{min}$) innerhalb der Saugleitung (37) unter den am Potentiometer (39) eingestellten Wert plus der kleinen Druckdifferenz ($\Delta$— $P_{min}$) abgesunken ist.

**Claims**

1. Suction device comprising a plurality of suction electrodes (36) for a suction massage during an electrotherapy with low-frequency or medium-frequency stimulation currents, wherein the suction electrodes are in connection with a vacuum pump (21) via a common suction line (37), wherein a ventilation device is connected with the suction line, by which the suction line can be vented during the application of the suction electrodes, and wherein for the suction massage a device for generating a pulsating suction is provided, characterized in that to the common suction line (37) a pressure sensor (32) is connected, which converts the value (— P) of the suction into an electrical voltage $u_p = f$ (— P), that an electrical control (33) having a first set-point transmitter (39) is provided, at which a desired limiting value of the suction for the application of the suction electrodes (36) can be set, that the electrical control (33) comprises a second set-point transmitter (38), at which a second limiting value of the suction can be set for the suction massage, and that the electrical control (33) is connected to the ventilation device (30, 31) so that the suction required both for the application and also for the suction massage can be limited or regulated to the set-point values set before the application of the suction electrodes.

2. Suction device comprising a plurality of suction electrodes (3) for a suction massage during an electrotherapy with low-frequency or medium-frequency stimulation currents, wherein the suction electrodes are in connection via a common suction line (15) with a vacuum pump (1), wherein a first valve device (4) is connected with the suction line, by which the suction line can be vented during the application of the suction electrodes, and wherein a second valve device (6) is connected with the suction line, by the actuation of which for the suction massage the suction can pulsate between a minimum and a maximum suction value, characterized in that, for an automatic limitation of the maximum suction ($-P_{max}$), during the application of the suction electrodes (3) and during the carrying-out of the suction massage, the first valve device (4) is constructed in such a manner making possible a reproducible presetting that, when the maximum suction ($-P_{max}$) is reached, it opens sufficiently far and for sufficient time for the flowing-in air to prevent a further rise in the suction beyond the set maximum suction ($-P_{max}$), and that for the automatic limitation of the minimum suction ($-P_{min}$) for the suction massage, a third valve device (5) is associated with the second valve device (6), at which (third valve device) the minimum suction ($-P_{min}$) for this suction massage can be preset.

3. Suction device according to Claim 1 or 2, characterized in that, between each suction electrode (3 ; 36) and the common suction line (15 ; 37) a throttle (14 ; 35) is provided, which throttle throttles the air stream sufficiently far that the suction in the common suction line (15 ; 37) is greater than the minimum suction — $P_{min}$ necessary for the application of the suction electrodes (3 ; 36) when no suction electrode (3 ; 36) has been applied to the patient.

4. Suction device according to one of the preceding Claims, characterized in that, for emptying a water separator (10 ; 25) provided between the suction electrodes (3 ; 36) and the vacuum pump (1 ; 21), a manullay actuated shut-off valve (26) and a solenoid valve (27, 28) are arranged in series, and that this solenoid valve (27, 28) is opened only when the suction device is switched off.

5. Suction device according to Claim 1, characterized in that an electrical measuring instrument (34) is connected to the pressure sensor (32), which measuring instrument is supplied from the output voltage $u_p = f (-P)$ of the pressure sensor and the scale of which is calibrated in pressure values.

6. Suction device according to Claim 1, characterized in that the electrical control (33) switches on the electric motor (22) of the vacuum pump (21) until a minimum suction ($-P_{min}$), which can be set for the application, has been reached, and switches it off as soon as this suction value has been reached, and that the electrical control (33), when the set minimum suction value is exceeded, opens the ventilating device constituted of a solenoid valve (30, 31) so that the common suction line (37) is vented via a throttle (29) sufficiently far for this set suction value to be reached and this solenoid valve (30, 31) closes again, as soon as this set suction value is reached.

7. Suction device according to Claim 6, characterized in that for the suction massage two potentiometers (38, 39) are provided as set point transmitters, and that the electrical control (33) switches on the electric motor (22) of the vacuum pump (21) until the maximum suction — $P_{max}$ is reached and, as soon as the maximum suction — $P_{max}$ is reached, switches off the electric motor (22) of the vacuum pump (21) and simultaneously opens the solenoid valve (30, 31) so that the suction electrodes (36) are vented until the previously set minimum suction — $P_{min}$ is reached and this solenoid valve (30, 31) immediately closes again as soon as the minimum suction — $P_{min}$ is reached and simultaneously again switches on the electric motor (22) of the vacuum pump (21), after which this cycle repeats periodically so that the suction periodically alternates between — $P_{min}$ and — $P_{max}$.

8. Suction device according to Claim 7, characterized in that a change-over switch (40) is present at the electrical control (33), at which switch this electrical control (33) can be alternatively set for the application of the suction electrodes (36) without suction massage at a first switching position (a) or for the suction massage at a second switching position (b).

9. Suction device according to one of Claims 6, 7 or 8, characterized in that the venting rate at the throttle (29) can be set, by which the period duration during the suction massage can be varied.

10. Suction device according to one of Claims 1, 5, 6 or 7, characterized in that in the common suction line (37) a solenoid valve (23) is disposed immediately behind the vacuum pump (21), which valve is opened only when the electric motor (22) of the vacuum pump (21) is switched on and which immediately closes when the electric motor (22) is switched off.

11. Suction device according to one of Claims 1, 6 or 7, characterized in that the electric motor (22) of the vacuum pump (21) is switched on and off via a triac (50), the firing of the triac (50) being effected from an electronic triac firing device (49), which always supplies firing pulses only when an operating alternating voltage ($U_3$) passes through the zero point and which also contains an optocoupler for potential separation between the operating alternating voltage and an operating voltage (U) for transistors (47, 48).

12. Suction device according to Claim 10, characterized in that the setting of the suction values — $P_{min}$ and — $P_{max}$ for the suction massage is effected via two separate potentiometers (38, 39) which are supplied from a constant voltage source ($U_1$), the electrical voltages tapped at the potentiometers (38, 39) being capable of being adjusted each by means of a voltage divider (62, 69 and 59, 66) to two variable values ($U_5$ and $U_2$), which are each independently supplied to the input of a first and second voltage comparator (44 and 42), where they are compared with the voltage ($U_p$) coming from the pressure sensor (32), which voltage also can be adjusted by means of a voltage divider (60, 67) to a variable voltage ($U_3$), the output signals $Q_{44}$, $Q_{42}$ of the comparators (44, 42) being furthermore supplied to an RS flip-flop, consisting of two NAND gates (45, 46), of which the output signals $Q_{45}$, $Q_{46}$ are represented as a function of the input singlas $Q_{44}$, $Q_{42}$ in the following matrix (70)

| $Q_{42}$ | $Q_{44}$ | $Q_{45}$ | $Q_{46}$ |
|---|---|---|---|
| 0 | 0 | 1 | 1 |
| 0 | 1 | 1 | 0 |
| 1 | 0 | 0 | 1 |
| 1 | 1 | / | / |

and wherein the output signals of the NAND gates govern via transistors (47, 48) the electric motor (22) of the vacuum pump (21), the solenoid valve (30, 31) for venting the suction line (37) and the solenoid valve (23, 24) for blocking the suction line (37) between the vacuum pump (21) and the water separator (25).

13. Suction device according the Claim 10, characterized in that the potentiometer (39) provided for the setting of the suction ($- P_{min}$) for the application of the suction electrodes (36) is supplied from a constant voltage source ($U_1$) and the output voltage of this potentiometer (39) can be adjusted, by means of resistors (61, 68), to a variable voltage ($U_4$), which is supplied to one input of a first comparator (43) and is compared with the voltage ($u_3$) coming from the pressure sensor (32), which is supplied to the second input of the comparator (43), the output signal ($Q_{43}$) of the comparator (43) governing, via a transistor (48), the electric motor (22) of the vacuum pump (21) and the solenoid valve (23, 24) which is disposed in the suction line (37) immediately behing the vacuum pump (21).

14. Suction device according to Claim 13, characterized in that, for the application of the suction electrodes, a second comparator (41) is connected in parallel with the first comparator (43), the input voltage ($U_1$) of which second comparator is adjusted by means of resistors (58, 65) to the somewhat smaller than the adjustable input voltage ($U_4$) of the first comparator (43), so that the output signal ($Q_{41}$) of the second comparator (41) flips from 0 to 1 when the suction ($- P_{min}$) in the suction line (37) rises by a small amount ($\Delta P_{min}$) above the value set at the potentiometer (39), with the result that the solenoid valve (30, 31) for venting the suction line is opened via a transistor (47)until the suction ($- P_{min}$) inside the suction line (37) has fallen below the value set at the potentiometer (39) plus the small pressure difference ($\Delta - P_{min}$).

## Revendications

1. Dispositif de succion avec plusieurs électrodes de succion (36) pour le massage par succion pendant une électrothérapie aux courants d'excitations à hautes et basses fréquences, au cours de laquelle les électrodes de succion sont reliées à une conduite de succion commune (37) au moyen d'une pompe à vide (21), auprès de laquelle un dispositif de ventilation est relié à la conduite de succion permettant l'aération de la conduite de succion lors de l'application des électrodes de succion, et auprès de laquelle a été prévu, pour le massage par succion, un dispositif générant une dépression vibratoire, caractérisé par le fait qu'un capteur de pression (32) est branché sur la conduite de succion commune (37) transformant la valeur de dépression ($- P$) en une tension électrique $U_p = f (- P)$ ; qu'une commande électrique (33) disposant d'un premier ajusteur de consigne (39) a été prévue, permettant le réglage de la valeur limite de dépression souhaitée pour l'application des électrodes de succion (36), que la commande électrique (33) dispose d'un deuxième ajusteur de consigne (38), permettant le réglage d'une deuxième valeur limite de la dépression pour le massage par succion, et que la commande électrique (33) est branchée sur le dispositif de ventilation permettant de la sorte à limiter ou à régler la dépression requise tant pour l'application que pour le massage par succion à partir des valeurs de consigne réglées avant l'application.

2. Dispositif de succion avec plusieurs électrodes de succion (3) pour un massage par succion lors d'une électrothérapie aux courants d'excitation à hautes et à basse fréquences, pendant laquelle les électrodes de succion sont reliées à une conduite de succion commune (15) au moyen d'une pompe à vide (1), auprès de laquelle un premier dispositif à soupapes (4) est relié à la conduite de succion permettant la ventilation de la conduite de succion lors de l'application des électrodes de succion, et auprès de laquelle un deuxième dispositif à soupapes (6) est relié à la conduite de succion, son actionnement pendant le massage par succion permettant à la dépression de pulser entre une dépression minimale et maximale, caractérisé par le fait que, pour atteindre une limitation automatique de la dépression maximum ($- P_{max}$), lors de l'application des électrodes de succion (3) et lors de l'exécution du massage par succion, le premier dispositif à soupapes (4), rendant possible un préréglage reproductible, soit conçu de façon à s'ouvrir assez loin et pendant assez longtemps lorsque la dépression maximale ($- P_{max}$) est atteinte, pour que l'air affluante empêche une augmentation de la dépression au-delà de la dépression maximum ($- P_{max}$) réglée, et que, pour obtenir une limitation automatique de la dépression minimale ($- P_{min}$) lors du massage par succion, un troisième dispositif à soupapes (5) est ajouté au deuxième dispositif à soupapes (6) permettant le préréglage de la dépression minimale ($-P_{min}$) nécessitée pour le massage par succion.

3. Dispositif de succion conforme aux revendications 1 et 2, caractérisé par le fait qu'un dispositif d'étranglement (14 ; 35) a été prévu entre chaque électrode de succion (3 ; 36) et la conduite de succion commune (15 ; 37), un dispositif d'étranglement qui étrangle l'écoulement d'air de telle manière que la dépression dans la conduite de succion commune (15 ; 37) soit plus élevée que la dépression minimale ($-P_{min}$) nécessaire à l'application des électrodes de succion (3 ; 36) lorsqu'aucune électrode de succion n'a été appliquée sur le malade.

4. Dispositif de succion suivant une des revendications précitées, caractérisé par le fait que, pour pouvoir procéder au vidage du séparateur d'eau (10 ; 25) prévu entre les électrodes de succion (3 ; 36) et la pompe à vide (1 ; 21), une soupape d'arrêt à commande manuelle (26) et une électrovanne (27 ; 28) ont été installés en série, et que l'électrovanne n'est ouverte que si le dispositif de succion est hors circuit.

5. Dispositif de succion conforme à la revendication 1, caractérisé par le fait qu'un appareil de mesurage (34) est branché sur le capteur de pression (32) et alimenté par la tension de sortie $U_p = f (- P)$ du capteur de pression avec étalonnage du cadran en valeurs de pression.

6. Dispositif de succion conforme à la revendication 1, caractérisé par le fait que la commande électrique (33) enclenche le moteur électrique (22) de la pompe à vide (21) et le garde enclenché jusqu'à ce que la dépression minimale ($-P_{min}$) réglable pour l'application est atteinte, et le coupe directement lorsque cette dépression est atteinte, et que, dès que la dépression minimale réglable est dépassée, la commande électrique (33) ouvre le dispositif de ventilation consistant en une électrovanne (30, 31) de sorte que la conduite de succion commune (37) soit ventilée à partir d'un étrangleur (29), et ce jusqu'au moment où la pression réglée est de nouveau atteinte et que cette électrovanne (30, 31) se referme de nouveau dès que ladite dépression réglée est atteinte.

7. Dispositif de succion conforme à la revendication 6, caractérisé par le fait que deux potentiomètres (38, 39) sont prévus comme ajusteurs de valeurs consigne pour le massage par succion, et que la commande électrique (33) enclenche le moteur électrique (22) de la pompe à vide (21) et le garde enclenché jusqu'à ce que la dépression maximale ($-P_{max}$) est atteinte et que, dès que celle-ci est atteinte, elle met le moteur électrique (22) de la pompe à vide (21) hors circuit et ouvre en même temps l'électrovanne (30, 31) de sorte que les électrodes de succion (36) soient ventilées aussi longtemps que nécessaire pour atteindre la dépression minimale $-P_{min}$ réglée au préalable, et que cette électrovanne (30, 31) se referme immédiatement dès que la dépression minimale $-P_{min}$ est atteinte, et qu'elle réenclenche en même temps le moteur électrique (22) de la pompe à vide (21), sur quoi ce cycle se répète périodiquement permettant ainsi à la dépression d'alterner périodiquement entre $-P_{min}$ et $-P_{max}$.

8. Dispositif de succion conforme à la revendication 7, caractérisé par le fait qu'auprès de la commande électrique (33) il existe un commutateur (40) au moyen duquel la commande électrique (33) peut être réglée alternativement pour l'application des électrodes de succion (36) sans massage par succion en une première position de commutation (a), ou pour un massage par succion en une deuxième position de commutation.

9. Dispositif de succion conforme à l'une des revendications 6, 7 ou 8, caractérisé par le fait que la vitesse de ventilation de l'étrangleur (29) peut être réglée permettant ainsi de varier la durée des périodes pendant le massage par succion.

10. Dispositif de succion conforme aux revendications 1, 5, 6 ou 7, caractérisé par le fait qu'une électrovanne (23) est implantée dans la conduite de succion (37) commune directement derrière la pompe à vide (21), qui n'est ouverte que lorsque le moteur électrique (22) de la pompe à vide (21) est enclenché et qui se referme directement lorsque le moteur électrique (22) est mis hors circuit.

11. Dispositif de succion conforme aux revendications 1, 6 ou 7, caractérisé par le fait que le moteur électrique (22) de la pompe à vide (21) est enclenché et mis hors circuit au moyen d'un triac (50), l'amorçage du triac (50) étant effectué au moyen d'un dispositif d'amorçage pour triacs (49) qui ne livre les impulsions d'amorçage que lors du passage par zéro de la tension alternative du secteur ($U_3$) et qui contient en plus un optocoupleur séparant les potentiels de la tension alternative du secteur et de la tension de service (U) pour transistors (47, 48).

12. Dispositifs de succion, conforme à la revendication 10, caractérisé par le fait que le réglage des dépressions $-P_{min}$ et $-P_{max}$ pour le massage par succion est effectué au moyen de deux potentiomètres séparés (38, 39) qui sont alimentés à partir d'une source de tension constante ($U_1$), les tensions électriques prises sur les deux potentiomètres pouvant toutes les deux être ajustées à deux valeurs (de tension) variables ($U_5$ et $U_2$) au moyen d'un diviseur de tension (62, 69 et 59, 66) ; ces dernières sont conduites, chacune pour soi, vers les entrées d'un premier et d'un deuxième comparateur de tension (44 et 42) où elles sont comparées à la tension ($U_p$) venant du capteur de pression (32) qui, elle aussi, peut être ajustée au moyen d'un diviseur de tension (60, 67) à une tension variable ($U_3$), les signaux de sortie $Q_{44}$, $Q_{42}$ des comparateurs (44, 42) étant en plus conduits vers une bascule électronique RS composée de deux conditionneurs inverseurs (45, 46) dont les signaux de sortie $Q_{45}$, $Q_{46}$ sont représentés en fonction des signaux d'entrée $Q_{44}$, $Q_{42}$ à l'aide de la matrice suivante (70),

| $Q_{42}$ | $Q_{44}$ | $Q_{45}$ | $Q_{46}$ |
|:---:|:---:|:---:|:---:|
| 0 | 0 | 1 | 1 |
| 0 | 1 | 1 | 0 |
| 1 | 0 | 0 | 1 |
| 1 | 1 | / | / |

les signaux de sortie des conditionneurs inverseurs commandant, par l'intermédiaire de transistors, (47, 48) le moteur électrique (22) de la pompe à vide (21), l'électrovanne (30, 31) pour la ventilation de la conduite de succion (37) et l'électrovanne (23, 24) pour la fermeture de la conduite de succion (37) entre la pompe à vide (21) et le séparateur d'eau (25).

13. Dispositif de succion, conforme à la revendication 10, caractérisé par le fait que le potentiomètre (39) prévu pour le réglage de la dépression ($-P_{min}$) nécessitée pour l'application des électrodes de succion (36) sont alimentés à partir d'une source de tension constante ($U_1$) et que la tension de sortie de ce potentiomètre (39) peut être réglée au moyen de résistances (61, 68) à une tension variable ($U_4$) qui est conduite sur une entrée du premier comparateur (43) et qui est comparée avec la tension ($U_3$) venant du capteur de pression (32), qui est conduite sur la deuxième entrée du comparateur (43), le signal de sortie ($Q_{43}$) du comparateur (43) commandant, par l'intermédiaire d'un transistor, (48) le moteur électrique (22) de la pompe à vide (21) ainsi que l'électrovanne (23, 24) implantée dans la conduite de succion (37), directement derrière la pompe à vide (21).

14. Dispositif de succion, conforme à la revendication 10, caractérisé par le fait que, pour l'application des électrodes de succion, un deuxième comparateur (41) a été branché en parallèle sur le premier comparateur (43), la tension d'entrée de ce deuxième comparateur ($U_1$) étant réglée à une valeur légèrement inférieure à celle de la tension d'entrée réglable ($U_4$) du premier comparateur (43), de sorte que le signal de sortie ($Q_{41}$) du deuxième comparateur bascule de 0 à 1 lorsque la dépression ($-P_{min}$) dans la conduite de succion (37) augmente d'une valeur ($\Delta P_{min}$) dépassant de très peu celle réglée au moyen du potentiomètre (39), de sorte que l'électrovanne (30, 31) pour la ventilation de la conduite de succion est ouverte par l'intermédiaire d'un transistor (48), et ce jusqu'à ce que la dépression ($-P_{min}$) à l'intérieur de la conduite de succion (37) soit descendue en-dessous de la valeur réglée au moyen du potentiomètre (39) plus la petite différence de pression ($\Delta -P_{min}$).

Fig.1

Fig.2

0 103 793

Fig.3

Fig.7

Fig.6

Fig.5

Fig.4